(19) Europäisches Patentamt

European Patent Office

Office européen des brevets

(11) Publication number: **0 294 814 B1**

# EUROPEAN PATENT SPECIFICATION

(45) Date of publication of patent specification: **12.01.94**  (51) Int. Cl.⁵: **A61K  31/415**

(21) Application number: **88109227.4**

(22) Date of filing: **09.06.88**

(54) **Treating agents for renal diseases.**

(30) Priority: **10.06.87 JP 144779/87**

(43) Date of publication of application:
**14.12.88 Bulletin  88/50**

(45) Publication of the grant of the patent:
**12.01.94 Bulletin  94/02**

(84) Designated Contracting States:
**AT BE CH DE ES FR GB GR IT LI NL SE**

(56) References cited:
**US-A- 4 665 188**

**JAP. J. NEPHROLOGY, vol. 24, no. 6, 1987,
pages 655-665; T. NIWA et al.: "Thromboxane
A2 metabolism and clinical effects of selec-
tive thromboxane A2 synthetase inhibitor in
patients with chronic glomerulonephritis"**

(73) Proprietor: **DAIICHI PHARMACEUTICAL CO.,
LTD.
14-10, Nihonbashi 3-chome
Chuo-ku, Tokyo 103(JP)**

(72) Inventor: **Masumura, Hidemi Daiichi Seiyaku
Research Inst.
16-13, Kitakasai 1-chome
Edogawa-ku
Tokyo(JP)**
Inventor: **Irie, Kiyoshi Daiichi Seiyaku Re-
search Inst.
16-13, Kitakasai 1-chome
Edogawa-ku
Tokyo(JP)**
Inventor: **Ashida, Shinichiro Daiichi Seiyaku
Research Inst.
16-13, Kitakasai 1-chome
Edogawa-ku
Tokyo(JP)**

(74) Representative: **Kinzebach, Werner, Dr. et al
Patentanwälte
Reitstötter, Kinzebach und Partner
Sternwartstrasse 4
Postfach 86 06 49
D-81633 München (DE)**

EP 0 294 814 B1

**Description**

This invention relates to the use of 6-(1-imidazolylmethyl)-5,6,7,8-tetrahydronaphthalene-2-carboxylic acid (hereinafter referred to as Compound A) or a pharmaceutically acceptable salt thereof for preparing a pharmaceutical composition for preventing and treating renal diseases, selected from diabetic nephropathy, hydronephrosis and ischemic renal insufficiency.

Compound A is known to inhibit the synthesis of thromboxane $A_2$ and have therapeutic effects on ischemic heart diseases (U.S. Patent 4,665,188). However, a therapeutic effect on renal diseases thereof has not been reported.

(E)-3-(4-(1-Imidazolylmethyl)phenyl)propenoic acid hydrochloride is known to inhibit the synthesis of thromboxane $A_2$. This compound was applied to diabetic nephropathy or glomerulonephritis (Abstract of Japanese Society of Nephrology 196 (1984) and Japanese Journal of Nephrology 24, 6, 655-665 (1987)), but the effect obtained was not satisfactory for the treatment of renal diseases.

The inventors have conducted extensive investigations on pharmacological activities of Compound A. As a result, it has now been found that Compound A exhibits excellent prophylactic and therapeutic effects on certain renal diseases.

This invention relates to the use of 6-(1-imidazolylmethyl)-5,6,7,8-tetrahydronaphthalene-2-carboxylic acid or a pharmaceutically acceptable salt thereof for preparing a pharmaceutical composition for preventing and treating renal diseases, selected from diabetic nephropathy, hydronephrosis and ischemic renal insufficiency.

Pharmaceutically acceptable salts of Compound A include acid addition salts formed with inorganic acids, e.g., hydrochloric acid, sulfuric acid and nitric acid, or organic acids, e.g., fumaric acid, tartaric acid, maleic acid, succinic acid, oxalic acid and benzenesulfonic acid and salts formed from a carboxyl group and an alkali metal, e.g., sodium and potassium or an alkaline earth metal, e.g., calcium and magnesium.

Compound A and a salt thereof are effective on various renal diseases. In particular, they can be suitably applied to treatments of diseases causing renal disorders, selected from diabetic nephropathy, obstructive nephropathy (hereinafter referred to as hydronephrosis) and ischemic renal insufficiency.

The acute toxicities ($LD_{50}$) of the hydrochloride hemihydrate of Compound A to male rats were 2438 mg/kg (p.o.) and 807 mg/kg (i.v.). Thus, Compound A is of very high safety.

Compound A can be formulated into various dose forms, such as tablets, powders, capsules, and injectable solutions, according to known pharmaceutical techniques and is usually administered orally, subcutaneously or intravenously.

An example of a preparation containing Compound A is described below.

| Tablets | |
| --- | --- |
| Hydrochloride hemihydrate of Compound A | 20 mg |
| Lactose | 50 mg |
| Corn Starch | 25.5 mg |
| Hydroxypropyl cellulose | 4 mg |
| Magnesium stearate | 0.5 mg |
| Total | 100 mg per one tablet |

The dose level of Compound A generally ranges from 30 to 1,000 mg/day for adult (body weight: about 50 to 60 kg) in oral administration.

Compound A experimentally exhibited excellent therapeutic effects on renal disorders in a diabetic nephropathy model, a hydronephrosis model and an ischemic renal insufficiency model and also exhibited excellent prophylactic and therapeutic effects on these diseases. Therefore, Compound A is useful as a preventing and treating agent for renal diseases.

The present invention is now illustrated in greater detail with reference to the following Test Examples.

TEST EXAMPLE 1

Efficacy in Diabetic Nephropathy Model

Test Animal:

5-Week old spontaneous hypertensive male rats (SHR) and normotensive Wister Kyoto rats (WKY), both available from Nippon Charles River, were used. Streptozotocin (STZ) was dissolved in a 0.1M citrate buffer solution (pH 4.5) and injected to the tail vein of the rat at a dose level of 50 mg/kg to prepare a diabetic rat. For control, the rat received 1 ml/kg of the citrate buffer solution alone through administration to the tail vein. After one week from the STZ administration, blood was taken from the tail vein of the unanesthetized rat, and the blood sugar level was measured. Those rats having a blood sugar level of 300 mg/kg or higher were used as diabetic rats.

Administration of Drug:

The hydrochloride hemihydrate of Compound A was dissolved in distilled water and administered orally to the test animal at a dose of 10 mg/kg/day for consecutive 5 months from one week after the administration of STZ or a citrate buffer solution.

Determination of Protein, $\gamma$-Glutarmyl Transpeptidase ($\gamma$-GTP) Activity and N-Acetyl-$\beta$-Glucosamidase (NA$\beta$G) Activity in Urine:

After 4 months from the start of the administration of Compound A, urine was collected for 24 hours, and the protein, $\gamma$-GTP activity and NA$\beta$G activity in the urine were determined by the method of Kinsbury et al., the method of using a commercially available kit ($\gamma$-GTP Test Pack, produced by Sankyo Co., Ltd.), and the method of Hasebe et al., respectively.

Serochemical Assay:

On the day next to the day of the final administration of Compound A, a blood sample was collected from the carotid artery under anesthesia with pentobarbital, and the blood was assayed by means of an automatic analyzing apparatus ("Hitachi Model 736", manufactured by Hitachi, Ltd.).

Test Results:

Table 1

| Inhibitory Activity on Increase of Excretion of Protein, $\gamma$-GTP and NA$\beta$G in the Urine in Diabetic SHR | | | | | |
|---|---|---|---|---|---|
| Test Animal | Dose (mg/kg/day) | Number of Animal | Excretion in Urine for 24 h | | |
| | | | Protein (mg/100 g B.W.) | $\gamma$-GTP (U/100 g B.W.) | NA$\beta$G (mg/100 g B.W.) |
| SHR | 0 | 8 | 2.79± 0.07 | 2.22± 0.14 | 0.332± 0.012 |
| " | 1 | 8 | 2.96± 0.09 | 2.70± 0.22 | 0.354± 0.006 |
| " | 10 | 9 | 2.74± 0.13 | 2.11± 0.11 | 0.313± 0.013 |
| Diabetic SHR | 0 | 7 | 7.74± 0.57 | 3.71± 0.23 | 0.946± 0.121 |
| " | 1 | 7 | 6.82± 1.56 | 2.83± 0.32* | 0.536± 0.086* |
| " | 10 | 8 | 4.00± 0.59 | 2.45± 0.22** | 0.412± 0.056** |

Note:
*$P < 0.05$,
**$P < 0.01$ (compared with control)
B.W. = Body weight

3

As is apparent from Table 1, Compound A significantly inhibits an increase in excretion of protein, $\gamma$-GTP and NA$\beta$G in the urine, which are indices for renal disorders.

## Table 2

### Influence on Body Weight of SHR and Diabetic SHR

| Test Animal | Dose (mg/kg/day) | | Body Weight (g) (Mean±S.E.) | | |
| --- | --- | --- | --- | --- | --- |
| | | | Before Admin. | After Start of Admin. | |
| | | | | 1 Month | 5 Months |
| SHR | 0 | (n=8) | 151.3±3.4 | 288.3±2.6 | 393.5±3.5 |
| " | 1 | (n=8) | 147.1±1.5 | 280.3±4.0 | 392.4±5.6 |
| " | 10 | (n=9) | 155.2±2.2 | 278.7±5.3 | 385.3±7.5 |
| Diabetic SHR | 0 | (n=7) | 137.3±1.4 | 194.3±12.3 | 266.0±14.3 |
| " | 1 | (n=7) | 144.3±3.9 | 239.1±8.1[*] | 325.4±12.2[*] |
| " | 10 | (n=8) | 147.3±1.3 | 239.4±7.0[**] | 335.9±9.3[**] |

Note:  *P < 0.05, **P < 0.01 (compared with a control group having received no drug)

n = Number of test animals

S.E. = Standard error

As is apparent from Table 2, Compound A alleviates inhibition on body weight grain due to renal disorders in SHR.

It can be seen from these results that Compound A possesses therapeutic effects on diabetic nephropathy and renal disorders caused by this disease.

TEST EXAMPLE 2

Efficacy in Hydronephrosis

Test Animal:

An abdominal incision was made in a SD male rat having a body weight of from 300 to 400 g under light anesthesia with diethyl ether. After the left urinary tract was ligated in the middle between the bladder and the kidney, the incision was closed, and the animal was fed on foods and tap water, ad lib.

Clearance Test:

After 18 hours from the ligation of the urinary tract, a cannula was inserted in the urinary tracts on both sides, the right femoral artery and the right femoral vein for collection of blood samples, infusion of inulin and infusion of p-aminohippuric acid, respectively, under Inactin anesthesia. Physiological saline containing 20 mg/ml of inulin and physiological saline containing 4 mg/ml of p-aminohippuric acid were continuously infused each at a rate of 0.4 ml/min in a 1/20 amount of the body weight. On confirming that the urinary output became constant after 50 minutes from the start of the infusion, urine was collected separately from the right and left urinary tracts at intervals of 20 minutes, and blood samples were obtained at the middle between urine collections. Immediately preceding the start of urine collection, infusion of physiological saline at a rate of 25 $\mu$l/min into the left femoral artery was instituted and continued for 40 minutes.

Thereafter, a solution of a hydrochloride hemihydrate of Compound A in physiological saline was continuously infused at a dose level of 0.3 $\mu$g/25 $\mu$l/min/rat for 40 minutes into the inlet of the renal artery through a retrograde cannula inserted from the left femoral artery. Inulin levels in urine and in blood were determined according to the anthrone method (H.H. Hilger et al., Pflügers Arch Physiol., Vol. 267, 218 (1958)), and p-aminohippuric acid levels in urine and blood were determined according to the method of Smith et al. (H.W. Smith et al., J. Clin. Invest., Vol. 24, 388 (1945)), to calculate clearance values of inulin and p-aminohippuric acid.

Test Results:

## Table 3

## Influence on Functions of Hydronephrotic Kidney

|  | Dose ($\mu$g/min) | Urinary Output ($\mu\ell$/min/ kidney) | Inulin Clearance (ml/min/ kidney) | p-Aminohippuric Acid Clearance (ml/min/kidney) |
|---|---|---|---|---|
| Normal Kidney | 0 | 7.76± 1.23 | 1.51± 0.11 | 5.03±0.51 |
| " | 0.3 | 7.47± 0.83 | 1.77± 0.13 | 5.95±0.50 |
| Hydronephrotic Kidney | 0 | 1.26± 0.15 | 0.16± 0.03 | 1.16±0.29 |
| " | 0.3 | 1.82± 0.21[*] | 0.34± 0.06[*] | 2.43±0.34[*] |

Note: Each value is a mean±S.E. of 5 cases.

*P < 0.05 (compared with control hydronephrotic kidney)

As can be seen from Table 3, the hydronephrotic kidney suffers serious reduction of inulin and p-aminohippuric acid clearance values as compared with the normal kidney, but such reduction can be improved by administration of Compound A. It was, therefore, proved that Compound A possesses excellent therapeutic effects on hydronephrosis and renal disorders caused by this disease.

TEST EXAMPLE 3

Efficacy in Ischemic Renal Insufficiency Model

Test Animal:

An abdominal incision was made in a SD vale rat weighing 300 to 450 g under pentobarbital anesthesia, and renal arteries of both sides were ligated with a clamp for 40 minutes and then reopened to prepare an animal suffering from isochemic renal insufficiency.

Administration of Drug:

The hydrochloride hemihydrate of Compound A was dissolved in physiological saline, and administered intraperitoneally to the test animal at a dose level of 10 mg/2 ml/kg 30 minutes before the ligature of the renal arteries. The control group received the same volume of physiological saline.

Determination of Renal Function:

After the blood flow of the renal arteries was restored, the animal was fed on foods and tap water ad lib. for 24 hours. The clearance values of inulin and p-aminohippuric acid were determined in the same manner as described in Example 2.

Test Results:

Table 4

| Influence on Functions of Ischemic Insufficient Kidney | | | | |
|---|---|---|---|---|
| Dose (mg/kg) | Number of Animals | Urinary Output ($\mu$l/min/kidney) | Inulin Clearance (ml/min/kidney) | p-Aminohippuric Acid Clearance (ml/min/kidney) |
| 0 | 6 | 8.10± 1.30 | 0.15± 0.03 | 0.40±0.09 |
| 10 | 8 | 11.30± 1.53* | 0.32± 0.05* | 1.01±0.17* |
| Note: Each value is a means±S.E. | | | | |

*$P < 0.05$ (Compared with control kidney)

AS is shown in Table 4, Compound A improves renal disorders in an ischemic renal insufficiency model, proving that it is useful for prevention and treatment of ischemic renal insufficiency and renal disorders caused by this disease.

TEST EXAMPLE 4

Acute toxicities of a hydrochloride hemihydrate of Compound A in rats through oral administration or intravenous injection were as follows.

Table 5

| Acute Toxicity in Rats | |
|---|---|
| $LD_{50}$ (mg) | |
| Male | Female |
| 2438 | 1994 (p.o.) |
| 807 | 783 (i.v.) |

**Claims**

1. The use of 6-(1-imidazolylmethyl)-5,6,7,8-tetrahydronaphthalene-2-carboxylic acid or a pharmaceutically acceptable salt thereof for preparing a pharmaceutical composition for preventing and treating renal diseases, selected from diabetic nephropathy, hydronephrosis and ischemic renal insufficiency.

**Patentansprüche**

1. Verwendung von 6-(1-Imidazolylmethyl)-5,6,7,8-tetrahydronaphthalin-2-carbonsäure oder eines pharmazeutisch akzeptablen Salzes davon zur Herstellung eines pharmazeutischen Mittels zur Verhinderung und zur Behandlung von Nierenerkrankungen, ausgewählt unter diabetischer Nephrophathie, Hydronephrose und ischemischer Niereninsuffizienz.

**Revendications**

1. Emploi d'acide 6-(1-imidazolylméthyl)-5,6,7,8-tétrahydronaphtalène-2-carboxylique ou d'un sel pharmaceutiquement acceptable de ce dernier pour préparer une composition pharmaceutique destinée à prévenir et traiter des pathologies rénales sélectionnées parmi la néphropathie diabétique, l'hydronéphrose et l'insuffisance rénale ischiémique.